# EUROPEAN PATENT APPLICATION

(11) **EP 4 635 454 A1**
(43) Date of publication of application: **22.10.2025**
(21) Application number: 25169795.9
(22) Date of filing: 10.04.2025
(51) Int. Cl.: A61F 2/58, A61F 2/68

(54) **A PROSTHETIC HAND AND ASSOCIATED METHOD**

(30) Priority: 19.04.2024 GB 202405520
(71) Applicant: Open Bionics Ltd, Bristol BS1 2NB (GB)
(72) Inventor: WOOD, Steve, Bristol, BS1 2NB (GB)
(74) Representative: Haseltine Lake Kempner LLP

(57) **Abstract**

According to an aspect, there is provided a prosthetic hand comprising:
a wrist attachment part configured for connecting to a wrist connector;
a palm part, the palm part and wrist attachment part being rotatably coupled together to form at least part of a wrist joint; and
at least one locking member configured to selectively engage a corresponding engagement part coupled to the palm part, wherein the locking member is movable between an unlocked position, in which the locking member and engagement part are disengaged and the palm part is free to rotate relative to the wrist attachment part, and a locked position, in which the locking member and engagement part are lockingly engaged and the palm part is rotatably locked relative to the wrist attachment part,
wherein an end face of the locking member is configured to be flush with an end face of the wrist attachment part when the locking member is in the locked position and the end face of the locking member is configured to be proud of the end face of the wrist attachment part when the locking member is in the unlocked position.

## Description

### FIELD OF THE INVENTION

The present disclosure relates to a prosthetic hand and associated method, and particularly, although not exclusively, relates to a prosthetic hand with a lockable wrist joint.

### BACKGROUND OF THE INVENTION

It is known to provide a prosthetic hand that can connect to a wrist connector, which may, for example, be provided at the distal end of a prosthetic forearm (or portion thereof). An example of a wrist connector is the United States Manufacturing Company (USMC) style quick disconnect wrist, such as that disclosed in US3798680 and at https://fillauer.com/products/quick-disconnect-wrist. The USMC style quick disconnect wrist is configured to connect to a prosthetic hand in first and second configurations. In the first configuration, rotation of the prosthetic hand relative to the disconnectable wrist connector may be permitted. By contrast, in the second configuration, the prosthetic hand may be rotatably locked relative to the disconnectable wrist connector. The prosthetic hand is moved between the first and second configurations by moving the prosthetic hand between first and second axial positions relative to the disconnectable wrist connector respectively. In particular, the prosthetic hand may be axially spaced further from the wrist connector in the first configuration than in the second configuration.

It is desirable for a prosthetic hand to have an articulating wrist so that the position of a palm and fingers of the hand can be adjusted. The above-described wrist connector may facilitate selective rotation of the prosthetic hand about a longitudinal axis of the forearm. However, it is also desirable for a palm of the prosthetic hand to rotate about a lateral axis perpendicular to the longitudinal axis. It is further desirable for such rotation to be resisted so that the prosthetic hand may hold its adjusted position prior to locking.

The present invention seeks to provide such functionality in an ergonomic way.

### SUMMARY OF THE INVENTION

According to a specific aspect, there is provided a prosthetic hand comprising:
a wrist attachment part configured for connecting to a wrist connector;
a palm part, the palm part and wrist attachment part being rotatably coupled together to form at least part of a wrist joint; and
at least one locking member configured to selectively engage a corresponding engagement part coupled to the palm part, wherein the locking member is movable between an unlocked position, in which the locking member and engagement part are disengaged and the palm part is free to rotate relative to the wrist attachment part, and a locked position, in which the locking member and engagement part are lockingly engaged and the palm part is rotatably locked relative to the wrist attachment part.

An end face of the locking member may be configured to be flush with (e.g. level with) an end face of the wrist attachment part when the locking member is in the locked position. The end face of the locking member may be configured to be proud of (e.g. spaced apart from or extend beyond) the end face of the wrist attachment part when the locking member is in the unlocked position.

The engagement part may comprise a plurality of teeth. The locking member may selectively engage at least one of said teeth. The plurality of teeth may be arranged in an arc, e.g. a circular arc. The arc may be centred on an axis of rotation between the palm part and the wrist attachment part. The teeth may be shaped such that rotation of the engagement part may urge the locking member away from the locked position.

The locking member may be slidable with respect to the wrist attachment part. The locking member may be resiliently biased to the locked position. Alternatively, the locking member may be resiliently biased to the unlocked position. However, the locking member may not be resiliently biased to the unlocked or locked positions. The locking member may freely move between the unlocked and locked positions.

Movement of the locking member may be limited beyond the unlocked position. The prosthetic hand may further comprise a limiter configured to limit movement of the locking member beyond the unlocked position. A resilient element may extend across an opening in the locking member. The resilient element may abut a shoulder of the wrist attachment part to resiliently resist and limit movement of the locking member.

The locking member may engage an end face of the wrist connector. The locking member may have a substantially flat end face for engaging the wrist connector.

The prosthetic hand may comprise two locking members, e.g. disposed on opposite sides of the wrist attachment part. The prosthetic hand may comprise a corresponding engagement part for each locking member.

The prosthetic hand may comprise a resiliently biased member that successively engages one of a plurality of recesses as the palm part rotates relative to the wrist attachment part. The prosthetic hand may provide a plurality of discrete angular positions for the palm part relative to the wrist attachment part.

The wrist connector may be a United States Manufacturing Company (USMC) style quick disconnect wrist or any other quick release prosthetic connector that functions in a similar way.

The prosthetic hand may connect to the wrist connector in first and second configurations, in which the prosthetic hand may be in first and second axial positions relative to the wrist connector respectively. The wrist connector may be configured such that in the first configuration rotation of the prosthetic hand relative to the wrist connector may be permitted and in the second configuration the prosthetic hand may be rotatably locked relative to the wrist connector. **In** the first configuration, rotation of the prosthetic hand relative to the wrist connector may be permitted about an axis that is substantially perpendicular to the axis of rotation of the palm part relative to the wrist attachment part.

The locking member may be configured to engage the wrist connector such that the locking member may be unable to move to the unlocked position when the prosthetic hand is in the second axial position and the locking member may be able to move to the unlocked position when the prosthetic hand is in the first axial position.

The locking member may be configured to move from the unlocked position to the locked position as the prosthetic hand moves from the first axial position to the second axial position relative to the wrist connector.

An assembly may comprise the above-mentioned prosthetic hand and the wrist connector.

According to another specific aspect, there is provided a method for the above-mentioned prosthetic hand, the method comprising:
moving the prosthetic hand between the first and second axial positions relative to the wrist connector so that the palm part is selectively rotatably locked relative to the wrist attachment part of the prosthetic hand.

According to a specific aspect, there is provided a prosthetic foot comprising:
an ankle attachment part configured for connecting to an ankle connector;
a foot part, the foot part and ankle attachment part being rotatably coupled together to form an ankle joint; and
at least one locking member configured to selectively engage a corresponding engagement part coupled to the foot part, wherein the locking member is movable between an unlocked position, in which the locking member and engagement part are disengaged and the foot part is free to rotate relative to the ankle attachment part, and a locked position, in which the locking member and engagement part are lockingly engaged and the foot part is rotatably locked relative to the ankle attachment part. An end face of the locking member may be configured to be flush with (e.g. level with) an end face of the ankle attachment part when the locking member is in the locked position. The end face of the locking member may be configured to be proud of (e.g. spaced apart from or extend beyond) the end face of the ankle attachment part when the locking member is in the unlocked position.

These and other aspects will be apparent from and elucidated with reference to the embodiment(s) described hereinafter. Also, features described with respect to one aspect or embodiment may also be applied to other aspects or embodiments.

### BRIEF DESCRIPTION OF THE DRAWINGS

Exemplary embodiments will now be described, by way of example only, with reference to the following drawings, in which:
Figure 1 is a side view of a prosthetic hand according to an example of the present disclosure;
Figures 2a and 2b (collectively Figure 2) are side sectional views of the prosthetic hand with a locking member in an unlocked position according to the example of the present disclosure with Figures 2a and 2b showing a palm of the prosthetic hand in first and second positions respectively;
Figures 3a and 3b (collectively Figure 3) are side sectional views of the prosthetic hand with a locking member in a locked position according to the example of the present disclosure with Figures 3a and 3b showing a palm of the prosthetic hand in the first and second positions respectively;
Figures 4a and 4b (collectively Figure 4) are side sectional views of the prosthetic hand according to the example of the present disclosure with Figure 4a showing the prosthetic hand in a first axial position relative to a wrist connector and Figure 4b showing the prosthetic hand in a second axial position relative to the wrist connector;
Figures 5a and 5b (collectively Figure 5) are partial side sectional views of the prosthetic hand according to the example of the present disclosure with Figures 5a and 5b showing the locking member in unlocked and locked positions respectively;
Figure 6 is a partial front sectional view of the prosthetic hand according to the example of the present disclosure with the locking member in the locked position;
Figures 7a and 7b (collectively Figure 7) are partial front views of the prosthetic hand according to the example of the present disclosure with Figures 7a and 7b showing the locking member in unlocked and locked positions respectively;
Figure 8 is a bottom view of the prosthetic hand according to the example of the present disclosure; and
Figure 9 is a flowchart depicting a method according to the example of the present disclosure.

### DETAILED DESCRIPTION OF EMBODIMENTS

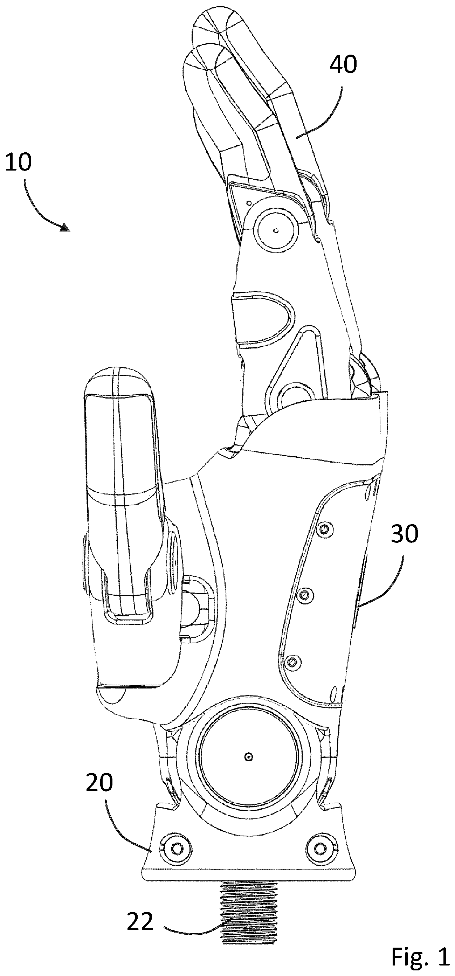

With reference to Figures 1 to 5, the present disclosure relates to a prosthetic hand 10 comprising a wrist attachment part 20 and a palm part 30. The prosthetic hand 10 may further comprise a plurality of digits 40, which may or may not rotate relative to the palm part 30.

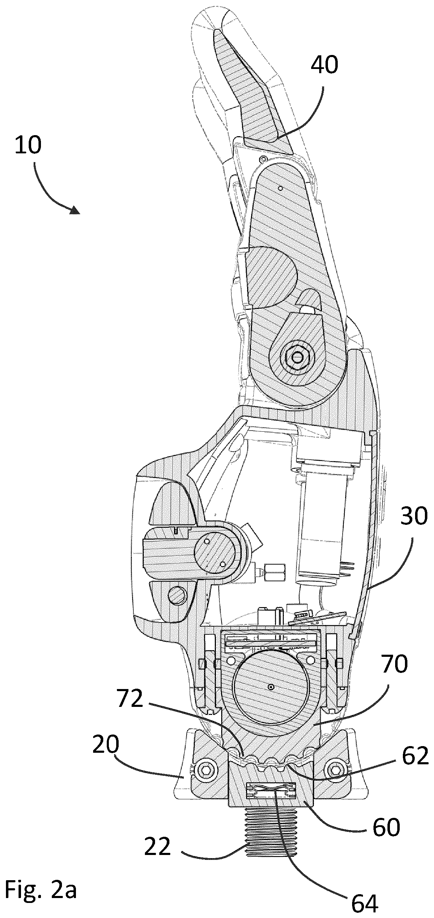

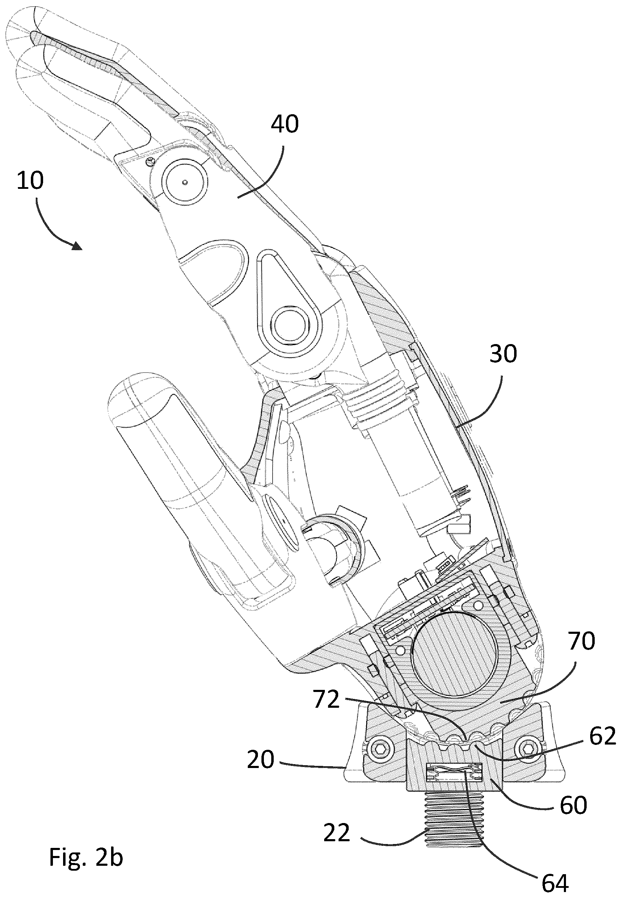

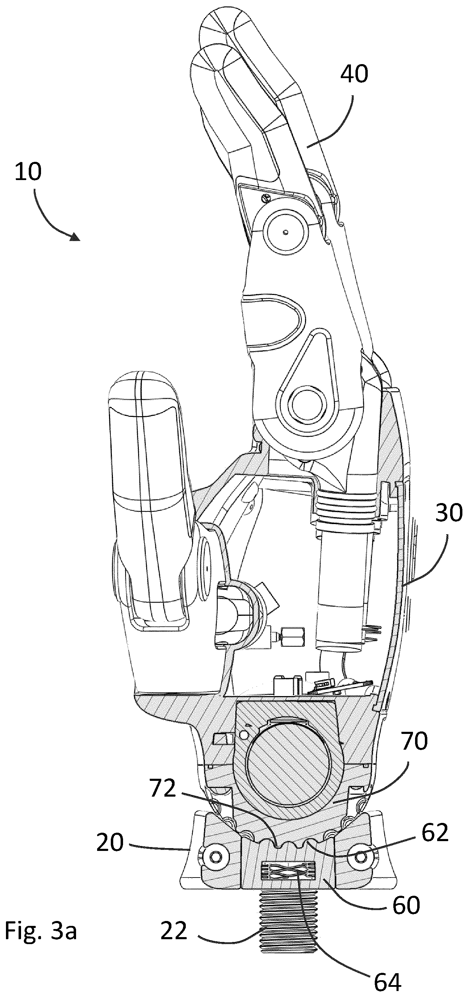

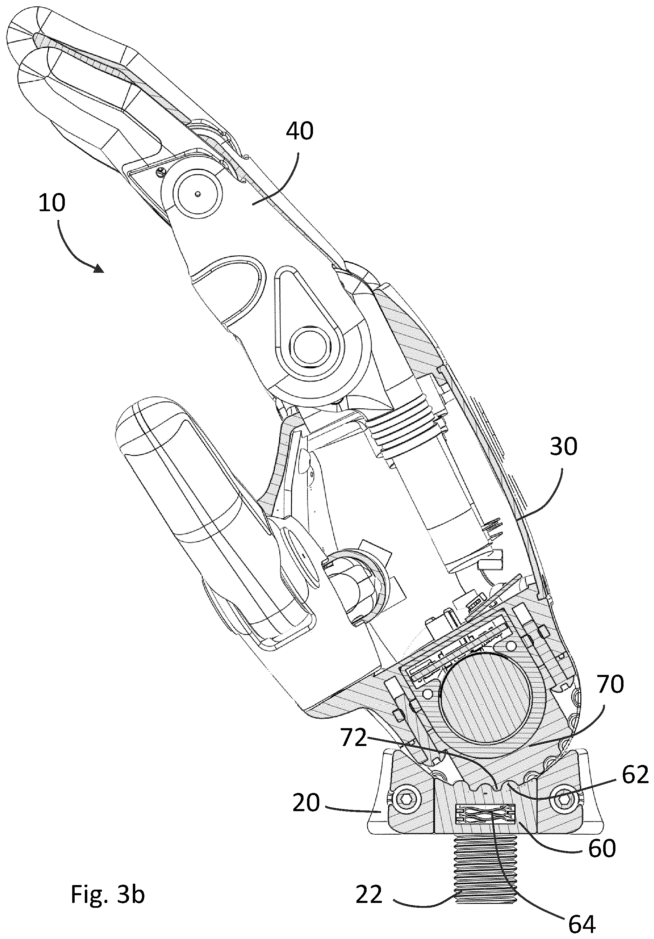

As depicted in Figures 2 and 3, the palm part 30 and wrist attachment part 20 are rotatably coupled together to form at least part of a wrist joint. The palm part 30 may rotate relative to the wrist attachment part 20 about a lateral axis that may be substantially perpendicular to a longitudinal axis of a forearm. Accordingly, the relative rotation of the palm part 30 and wrist attachment part 20 may permit extension and flexion of the prosthetic hand 10, e.g. relative to a user's forearm or prosthetic forearm. Figures 2a and 3a show the palm part 30 in a first position relative to the wrist attachment part 20, whereas Figures 2b and 3b shows the palm part 30 in a second position relative to the wrist attachment part 20.

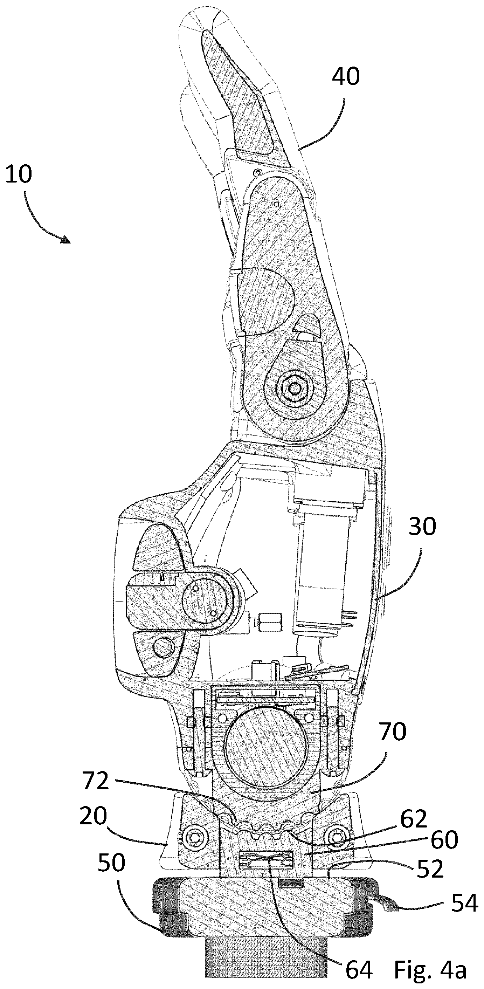

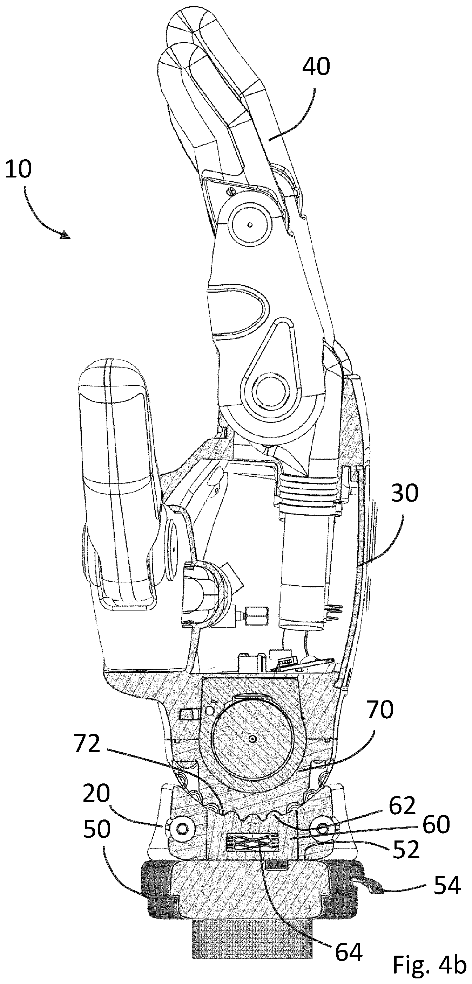

The wrist attachment part 20 is configured for connecting to a wrist connector 50, such as that shown in Figure 4. The wrist connector 50 may be provided at a distal end of a prosthetic arm or forearm. The wrist attachment part 20 may comprise a threaded shaft 22 that may engage the wrist connector 50. The threaded shaft 22 may engage the wrist connector 50, e.g. via an intermediate insert (not shown). As will be described in more detail below, the wrist connector 50 may be configured to receive the prosthetic hand 10 in one of two configurations. **In** a first configuration, the prosthetic hand 10 may be free to rotate about a longitudinal axis of the forearm, i.e. in supination and pronation directions. **In** a second configuration, the prosthetic hand 10 may be rotatably fixed relative to the wrist connector 50.

The prosthetic hand 10 further comprises at least one locking member 60 and at least one engagement part 70 for the locking member to engage. The engagement part 70 is coupled to the palm part 30 and may rotate with the palm part 30. The locking member 60 may be coupled, e.g. slidably coupled, to the wrist attachment part 20. The locking member 60 is configured to selectively engage the corresponding engagement part 70 coupled to the palm part, so as to prevent or restrict rotation of the palm part 30 relative to the wrist attachment part 20. **In** particular, the locking member 60 is movable between an unlocked position (shown in Figs 2, 4a and 5a), in which the locking member 60 and engagement part 70 are disengaged and the palm part 30 is free to rotate relative to the wrist attachment part 20, and a locked position (shown in Figs 3, 4b and 5b), in which the locking member 60 and engagement part 70 are lockingly engaged and the palm part 30 is rotatably locked relative to the wrist attachment part 20.

As depicted in Figures 2 to 5, the engagement part 70 may comprise a plurality of teeth 72. The plurality of teeth 72 may be arranged in an arc, e.g. a circular arc. The arc may be centred on an axis of rotation between the palm part 30 and the wrist attachment part 20. The engagement part 70 may thus form at least part of a gear. The locking member 60 may selectively engage at least one of said teeth 72, for example, the locking member 60 may also comprise at least one tooth 62 that engages meshes with a space between engagement part teeth 72. **In** the example shown, the locking member 60 comprises a plurality of teeth 62.

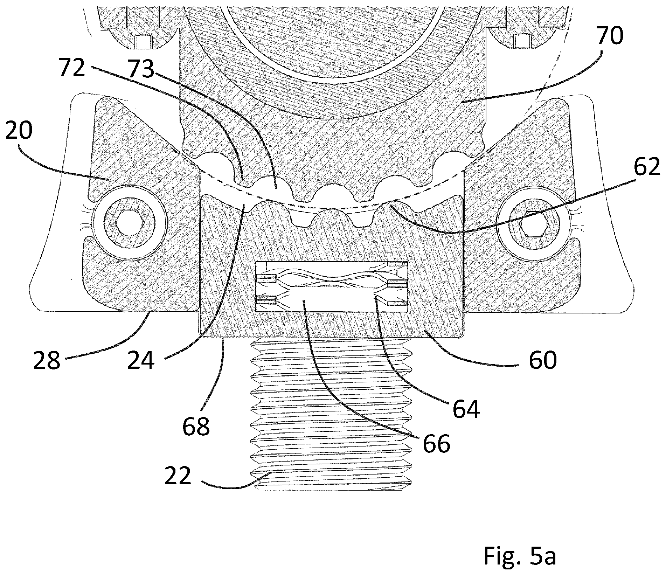

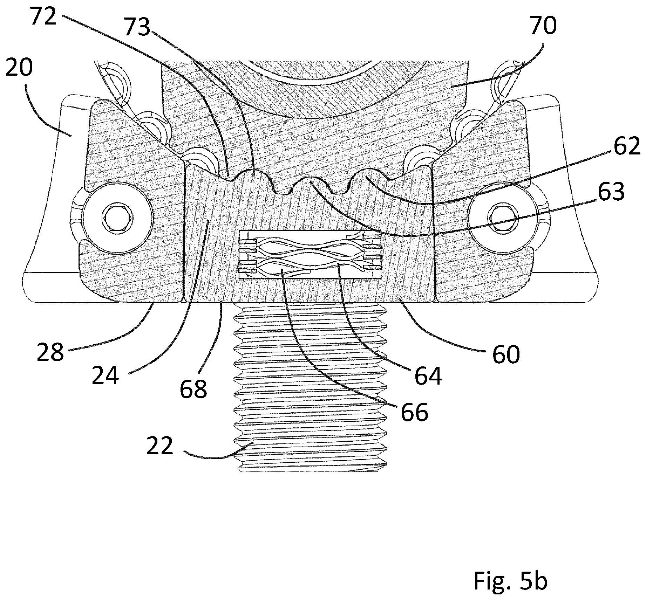

As is best shown in Figure 5, the engagement part teeth 72 may be shaped such that rotation of the engagement part 70 may urge the locking member 60 away from the locked position. For example, the engagement part teeth 72 may be formed by successive and substantially semi-circular recesses 73 in the engagement part 70. The locking member tooth (or teeth) 62 may be correspondingly shaped, e.g. with substantially semi-circular projections 63. The engagement part teeth 72 may be arranged on a convex surface of the engagement part 70. The locking member teeth 62 may be arranged on a concave surface of the locking member 60.

Referring still to Figure 5, the locking member 60 may be slidable with respect to the wrist attachment part 20. **In** particular, the locking member 60 may be slidably received in a channel 24 within the wrist attachment part 20. The locking member 60 may be resiliently biased towards the locked position, for example, by virtue of a resilient element 64. When the locking member 60 is free to move, the resilient element 64 together with the interaction between the engagement part teeth 72 and locking member tooth (or teeth) 62 may provide a plurality of discrete stable angular positions for the palm part 30 relative to the wrist attachment part 20. The palm part 30 may successively snap into each of the discrete angular positions as the palm part 30 is rotated. This conveniently allows the palm part 30 to hold its position prior to locking. This may help a user, who might otherwise be unable to hold the palm part in place.

Although, as described above, the locking member 60 may be resiliently biased into the locked position, it is also envisaged that the locking member may be resiliently biased to the unlocked position or not biased to either of the unlocked or locked positions (such that the locking member may freely move between the unlocked and locked positions). In this case, the prosthetic hand 10 may comprise a separate resiliently biased member that successively engages one of a plurality of detents or recesses as the palm part 30 rotates relative to the wrist attachment part 20. The prosthetic hand 10 may thus provide a plurality of discrete stable angular positions for the palm part 30 relative to the wrist attachment part 20. Alternatively, relative rotation between the palm part 30 and wrist attachment part 20 may be frictionally resisted such that the palm part 30 may hold its position relative to the wrist attachment part 20 when adjusted.

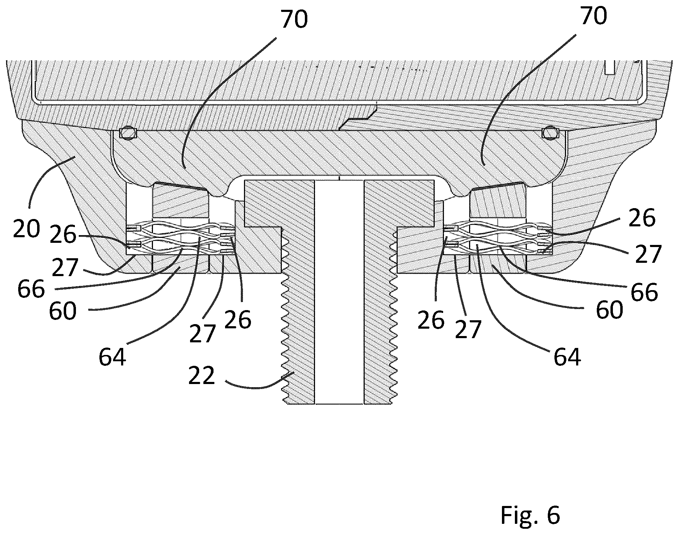

Referring to Figures 5 and 6, the resilient element 64 may be provided within a pocket or opening 66 within the locking member 60. The resilient element 64 may be a spring, such as a coil or wave spring. As shown in Figure 6, the resilient element 64 may extend beyond the opening 66 and into recesses 26 either side of the channel 24. The resilient element 64 may abut a shoulder 27 at an end of each recess 26. In this way, the resilient element 64 may limit movement of the locking member 60 such that it cannot move beyond the unlocked position. The resilient element 64 conveniently provides both the resilient resistance and limiting movement functionalities.

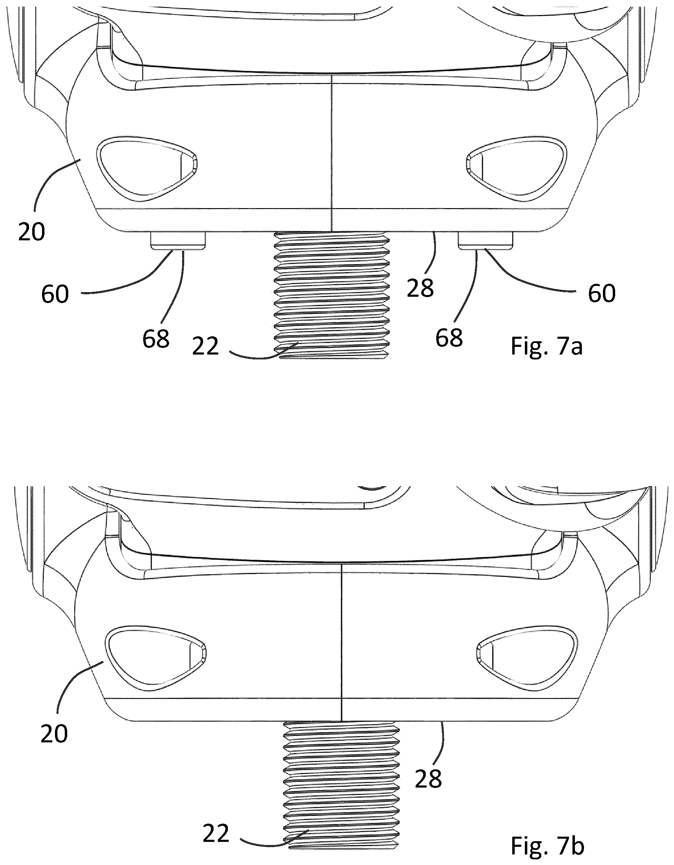

Turning now to Figure 7, the locking member 60 may comprise an end face 68 for selective engagement with the wrist connector 50. As depicted in Figure 7a, the locking member end face 68 may be proud of (e.g. spaced apart from or extend beyond) an end face 28 of the wrist attachment part 20 when the locking member 60 is in the unlocked position. By contrast, as depicted in Figure 7b, the locking member end face 68 may be configured to be flush with (e.g. level with) the wrist attachment part end face 28 when the locking member 60 is in the locked position. The locking member end face 68 and/or the wrist attachment part end face 28 may be substantially flat, but may otherwise be shaped to correspond to a shape of the wrist connector 50.

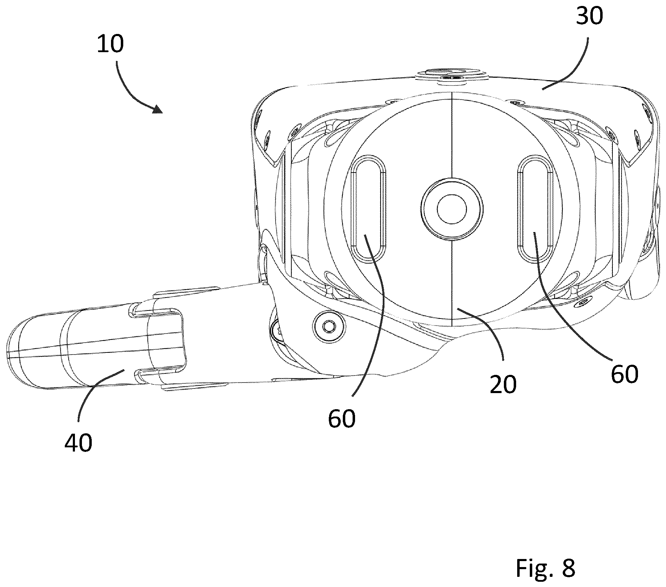

Referring to Figures 6, 7a and 8, the prosthetic hand 10 may comprise a pair of locking members 60, e.g. disposed on opposite sides of the wrist attachment part 20. The threaded shaft 22 may be provided between the locking members 60. It is also envisaged that other numbers of locking members may be provided. Features described above in respect of the locking member 60 may apply to each locking member, for example the prosthetic hand 10 may also comprise a pair of engagement parts 70 that engage corresponding locking members 60.

Returning to Figure 4, the wrist connector 50 may be configured such that the prosthetic hand 10 may connect to the wrist connector 50 in a first configuration (depicted in Figure 4a) and a second configuration (depicted in Figure 4b). In the first configuration, rotation of the prosthetic hand 10 relative to the wrist connector 50 may be permitted about an axis that is substantially parallel to a longitudinal axis of the forearm (e.g. in supination and pronation directions). In the second configuration, the prosthetic hand 10 may be rotatably locked relative to the wrist connector 50. By way of example, the wrist connector 50 may be a United States Manufacturing Company (USMC) style quick disconnect wrist similar to that disclosed in US3798680 or any other quick release prosthetic connector that functions in a similar way.

In the first configuration, the prosthetic hand 10 may be in a first axial position relative to the wrist connector 50. In the second configuration, the prosthetic hand 10 may be in a second axial position relative to the wrist connector 50. The prosthetic hand 10 (e.g. wrist attachment part end face 28) may be spaced apart from the wrist connector 50 (e.g. an end face 52 of the wrist connector 50) in the first axial position. The locking member 60 may be configured such that a distance travelled by the locking member 60 as it moves between the locked and unlocked positions is less than or equal to the spacing between the wrist attachment part end face 28 and the wrist connector end face 52 in the first axial position. By contrast, the wrist attachment part end face 28 may contact the wrist connector end face 52 in the second axial position.

An intermediate insert (e.g. that the threaded shaft 22 may connect to) may rotate in a bore of the wrist connector 50. The insert may slide between the first and second axial positions. In the second axial position, the insert may engage a spline or any other form of abutment to prevent rotation of the insert and thus prosthetic hand 10. The wrist connector 50 may also lock the prosthetic hand 10 in the second axial position such that the prosthetic hand may not be moved into the first axial position. The wrist connector 50 may comprise a release tab 54 that allows the prosthetic hand 10 to be released from the second axial position.

As the prosthetic hand 10 moves from the first axial position to the second axial position relative to the wrist connector 50, the locking member 60 may engage the end face 52 of the wrist connector 50 and the locking member 60 may be prevented from moving to the unlocked position. Rotation of the palm part 30 relative to the wrist attachment part 20 is thus prevented by the locking engagement of the locking members 60 with the engagement part 70. In its second configuration, the wrist connector 50 prevents rotation of the prosthetic hand 10 about the longitudinal axis of the forearm, and the consequential movement of the locking member 60 prevents rotation of the palm part 30 relative to the wrist attachment part 20. This allows a user to lock both degrees of freedom in a single convenient locking action that utilises the functionality of the wrist connector 50.

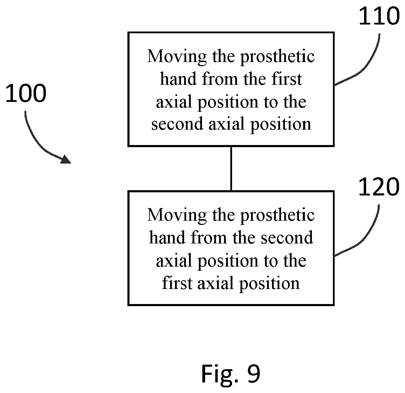

With reference to Figure 9, the present disclosure also relates to a method 100 for the above-described prosthetic hand 10. In a first action 110, the method 100 comprises moving the prosthetic hand 10 from the first axial position to the second axial position relative to the wrist connector 50 so that the palm part 30 is selectively rotatably locked relative to the wrist attachment part 20 of the prosthetic hand. In a second action 120, the method 100 comprises moving the prosthetic hand 10 from the second axial position to the first axial position relative to the wrist connector 50 so that the palm part 30 is rotatably unlocked relative to the wrist attachment part 20 of the prosthetic hand. A new position for the palm part 30 may then be selected before the palm part may be again locked into place.

Although the above description focusses on a prosthetic hand, it is also envisaged that the present invention may be applied to other terminal devices, such as a prosthetic foot. For example, locking members may engage an ankle connector to selectively lock the rotational position of a prosthetic foot as the prosthetic foot is moved between first and second axial positions relative to the ankle connector. The ankle connector may be provided at a distal end of a prosthetic leg or lower leg.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. Any reference signs in the claims should not be construed as limiting the scope.

For the avoidance of doubt, the disclosure extends to and includes the following numbered Clauses:
Clause 1. A prosthetic hand comprising:
   a wrist attachment part configured for connecting to a wrist connector;
   a palm part, the palm part and wrist attachment part being rotatably coupled together to form at least part of a wrist joint; and
   at least one locking member configured to selectively engage a corresponding engagement part coupled to the palm part, wherein the locking member is movable between an unlocked position, in which the locking member and engagement part are disengaged and the palm part is free to rotate relative to the wrist attachment part, and a locked position, in which the locking member and engagement part are lockingly engaged and the palm part is rotatably locked relative to the wrist attachment part,
   wherein an end face of the locking member is configured to be flush with an end face of the wrist attachment part when the locking member is in the locked position and the end face of the locking member is configured to be proud of the end face of the wrist attachment part when the locking member is in the unlocked position.
Clause 2. The prosthetic hand of Clause 1, wherein the engagement part comprises a plurality of teeth and the locking member selectively engages at least one of said teeth.
Clause 3. The prosthetic hand of Clause 2, wherein the plurality of teeth are arranged in an arc.
Clause 4. The prosthetic hand of Clause 1 or 2, wherein the teeth are shaped such that rotation of the engagement part urges the locking member away from the locked position.
Clause 5. The prosthetic hand of any of the preceding Clauses, wherein the locking member is resiliently biased to the locked position.
Clause 6. The prosthetic hand of any of the preceding Clauses, wherein the locking member is slidable with respect to the wrist attachment part.
Clause 7. The prosthetic hand of any of the preceding Clauses, wherein movement of the locking member is limited beyond the unlocked position.
Clause 8. The prosthetic hand of any of the preceding Clauses, wherein the locking member engages an end face of the wrist connector.
Clause 9. The prosthetic hand of any of the preceding Clauses, wherein the locking member has a substantially flat end face for engaging the wrist connector.
Clause 10. The prosthetic hand of any of the preceding Clauses, wherein the prosthetic hand comprises two locking members disposed on opposite sides of the wrist attachment part.
Clause 11. The prosthetic hand of any of the preceding Clauses, wherein the wrist connector is a United States Manufacturing Company (USMC) style quick disconnect wrist.
Clause 12. The prosthetic hand of any of the preceding Clauses, wherein the wrist connector is configured to connect to the prosthetic hand in first and second configurations, in which the prosthetic hand is in first and second axial positions relative to the wrist connector respectively.
Clause 13. The prosthetic hand of Clause 12, wherein the wrist connector is configured such that in the first configuration rotation of the prosthetic hand relative to the wrist connector is permitted and in the second configuration the prosthetic hand is rotatably locked relative to the wrist connector.
Clause 14. The prosthetic hand of Clause 12 or 13, wherein the locking member is configured to engage the wrist connector such that the locking member is unable to move to the unlocked position when the prosthetic hand is in the second axial position and the locking member is able to move to the unlocked position when the prosthetic hand is in the first axial position.
Clause 15. An assembly comprising the prosthetic hand of any of the preceding Clauses and the wrist connector.
Clause 16. A method for the prosthetic hand of any of Clauses 1 to 14, the method comprising:
   moving the prosthetic hand between the first and second axial positions relative to the wrist connector so that the palm part is selectively rotatably locked relative to the wrist attachment part of the prosthetic hand.

## Claims

1. A prosthetic hand comprising:
a wrist attachment part configured for connecting to a wrist connector;
a palm part, the palm part and wrist attachment part being rotatably coupled together to form at least part of a wrist joint; and
at least one locking member configured to selectively engage a corresponding engagement part coupled to the palm part, wherein the locking member is movable between an unlocked position, in which the locking member and engagement part are disengaged and the palm part is free to rotate relative to the wrist attachment part, and a locked position, in which the locking member and engagement part are lockingly engaged and the palm part is rotatably locked relative to the wrist attachment part,
wherein an end face of the locking member is configured to be flush with an end face of the wrist attachment part when the locking member is in the locked position and the end face of the locking member is configured to be proud of the end face of the wrist attachment part when the locking member is in the unlocked position.

2. The prosthetic hand of claim 1, wherein the engagement part comprises a plurality of teeth and the locking member selectively engages at least one of said teeth.

3. The prosthetic hand of claim 2, wherein the plurality of teeth are arranged in an arc.

4. The prosthetic hand of claim 1 or 2, wherein the teeth are shaped such that rotation of the engagement part urges the locking member away from the locked position.

5. The prosthetic hand of any of the preceding claims, wherein the locking member is resiliently biased to the locked position.

6. The prosthetic hand of any of the preceding claims, wherein the locking member is slidable with respect to the wrist attachment part.

7. The prosthetic hand of any of the preceding claims, wherein movement of the locking member is limited beyond the unlocked position.

8. The prosthetic hand of any of the preceding claims, wherein the locking member engages an end face of the wrist connector.

9. The prosthetic hand of any of the preceding claims, wherein the locking member has a substantially flat end face for engaging the wrist connector.

10. The prosthetic hand of any of the preceding claims, wherein the prosthetic hand comprises two locking members disposed on opposite sides of the wrist attachment part.

11. The prosthetic hand of any of the preceding claims, wherein the wrist connector is a United States Manufacturing Company (USMC) style quick disconnect wrist.

12. The prosthetic hand of any of the preceding claims, wherein the wrist connector is configured to connect to the prosthetic hand in first and second configurations, in which the prosthetic hand is in first and second axial positions relative to the wrist connector respectively and optionally wherein the wrist connector is configured such that in the first configuration rotation of the prosthetic hand relative to the wrist connector is permitted and in the second configuration the prosthetic hand is rotatably locked relative to the wrist connector.

13. The prosthetic hand of claim 12, wherein the locking member is configured to engage the wrist connector such that the locking member is unable to move to the unlocked position when the prosthetic hand is in the second axial position and the locking member is able to move to the unlocked position when the prosthetic hand is in the first axial position.

14. An assembly comprising the prosthetic hand of any of the preceding claims and the wrist connector.

15. A method for the prosthetic hand of any of claims 1 to 13, the method comprising:
moving the prosthetic hand between the first and second axial positions relative to the wrist connector so that the palm part is selectively rotatably locked relative to the wrist attachment part of the prosthetic hand.
